# EUROPEAN PATENT SPECIFICATION

(11) **EP 1 929 963 B1**
(45) Date of publication and mention of the grant of the patent: **17.02.2010**
(21) Application number: 06024997.6
(22) Date of filing: 04.12.2006
(51) Int. Cl.: A61B 17/072, A61B 17/22, A61B 17/28, A61B 17/32, A61B 19/00, A61B 17/00

(54) **Device for endoluminally or laparoscopically grasping and excising a tissue sample from areas in a patient's body**
Vorrichtung für das endoluminale oder laparoskopische Erfassen und Ausschneiden einer Gewebeprobe vom Körper eines Patienten
Dispositif pour saisir et exciser intraluminal ou laparoscopique un échantillon de tissu provenant d'un patient

(43) Date of publication of application: 11.06.2008
(73) Proprietor: ETHICON ENDO-SURGERY, INC., Cincinnati, OH 45242-2839 (US)
(72) Inventor: D'Arcangelo, Michele, 00142 Roma (IT); Kuhns, Jesse J., Cincinnatti, Ohio 45208 (US); Bilotti, Federico, 04011 Aprilia, Latina (IT)
(74) Representative: Leihkauf, Steffen Falk

(56) References cited:
- EP-A- 0 666 057
- WO-A-01/91646
- WO-A-97/47231
- WO-A-2006/027014
- US-A- 6 142 933
- US-B1- 7 118 528

## Description

The present invention concerns a device for endoluminally or laparoscopically grasping and excising a tissue sample from areas in a patient's body. In particular, the present invention concerns a device to be used in a method suitable for covering low and high gastrointestinal portions, laparoscopic approaches and more advanced transgastric, transvaginal or transanal/rectal approaches.

There is a great need for instruments for endoluminally or laparoscopically excising a tissue sample from areas in a patient's body so as to allow the safe and effective removal of a tissue sample, for example a lesion. In particular, there is a need for instruments for excising a determined and precise amount of tissue allowing better control of the visual field during each step of the method.

It is known to grasp and excise tissue samples by conventional open surgery. The disadvantages of conventional surgical methods are well known; for example, they are very invasive, the patient needs to be anaesthetised and the post-operation recovery time is long.

In order to overcome the drawbacks of the conventional open surgery approach, a device for endoluminally grasping and excising a tissue sample from areas in a patient's body has been proposed in the yet unpublished co-pending patent application MI2006A000411 by the same applicant. According to this endoluminal approach, a distal portion of a grasper is introduced into the patient's body and fixed to the tissue to be excised. A surgical stapling device is provided having a distal end with a window for receiving the tissue to be excised and comprising cutting and stapling means that act through that window, The surgical stapling device is introduced into the patient's body and its distal end is positioned close to the tissue to be excised. Then, traction is exerted on the traction means by which an amount of tissue is pulled through the window and positioned for cutting and stapling. After positioning of the amount of tissue, the latter is cut and stapled by actuating the cutting and stapling means of the surgical stapling device through the window.

US 7,118,528 B1 discloses a known surgical instrument assembly for the treatment of haemorrhoids and WO 01/91646 A1, which forms the basis of the two-part form of claim 1, discloses a surgical stapling instrument having a curved cartridge staple fastening assembly.

While the above described devices allow reducing the invasiveness of the surgical intervention, the limited size of the window of the surgical stapling device limits the amount of tissue that can be grasped and excised. Moreover, the surgical stapling device may at least partially obstruct the access to the tissue. As a consequence, an extensive articulation of the endoscope might be necessary in order to obtain a good visualization inside the window and to approach the tissue at the correct angle. Furthermore, the window configuration of the surgical stapling device requires the grasping and traction device to be inserted through the window opening and the visual field is partially occupied by the surgical stapling device, thereby hindering the visual detection (e.g. by an endoscope) of the most appropriate location for grasping the tissue.

It is therefore an object of the present invention to provide a device for endoluminally or laparoscopically excising a tissue sample enabling an improved visualization during detection and grasping the tissue.

Within the scope of the main object, another object of the present invention is to provide a device for endoluminally or laparoscopically excising a tissue sample enabling an increased amount of tissue to be grasped and excised.

Yet another object of the present invention is to provide a device for endoluminally excising a tissue sample carried out along the entire length of the colon up to the caecum.

These and other objects are obtained by a surgical device according to claim 1. The dependent claims cover embodiments of the invention.

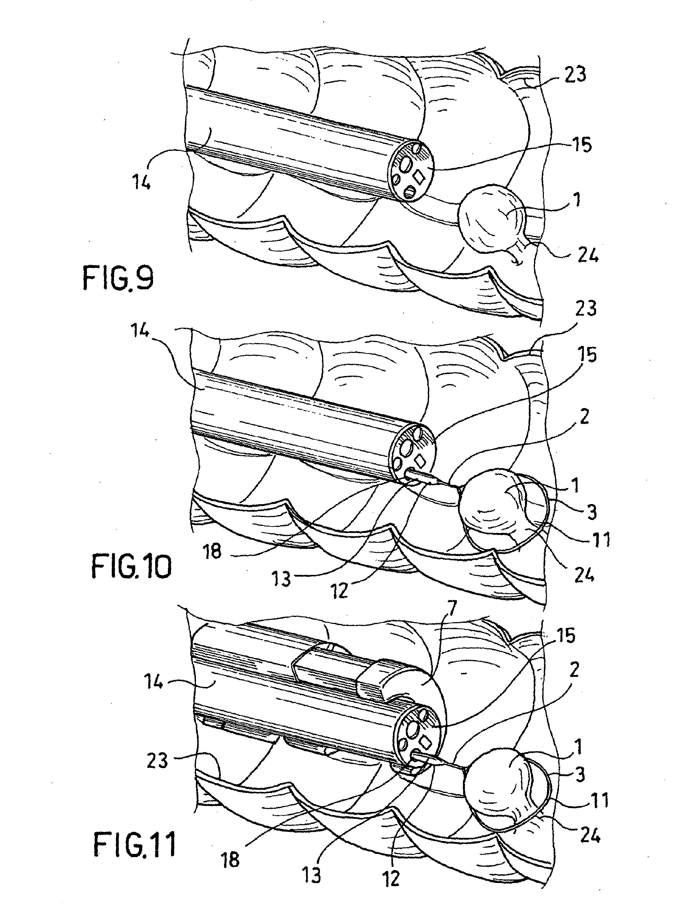

The details and advantages of the present invention shall be made apparent from the accompanying drawings and the description thereof, which illustrate embodiments of the invention and serve to explain the principles of the present invention.

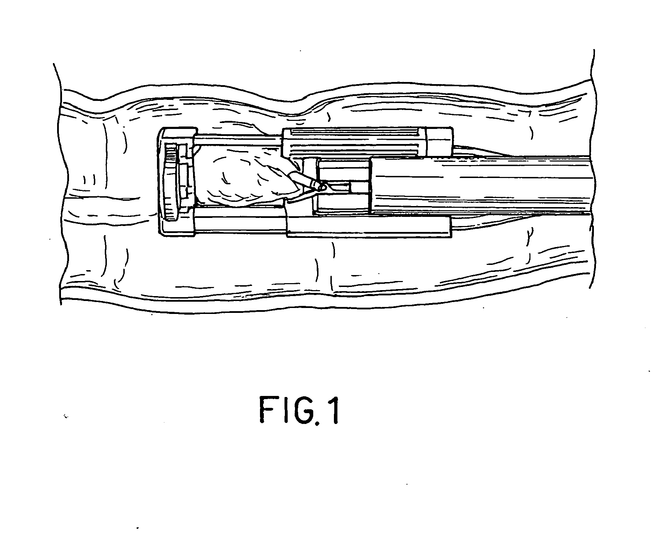

- Figure 1 shows a longitudinally sectioned portion of the colon and a surgical stapling device and a grasping device during the performance of the method for grasping and excising tissue described in the unpublished patent application MI2006A000411;
- Figure 2 is a perspective distal lateral view of a surgical stapling device according to the invention in an open configuration;
- Figure 3 is a perspective distal lateral view of the surgical stapling device in figure 2 in a closed configuration;
- Figure 4 is another perspective distal lateral view of the surgical stapling device in figure 2 in an open configuration;
- Figure 5 shows the surgical stapling device in figure 4 in a closed configuration;
- Figures 6, 7 and 8 are several side views of the surgical stapling device in an open configuration;
- Figures 9 to 14 show a longitudinally sectioned colon and a surgical staple device, a grasping device and an endoscope performing a method for endoluminally grasping and excising a tissue sample;
- Figures 15 to 19 show a longitudinally sectioned colon and a protective sheath, a grasping device and an endoscope performing several steps of a method for endoluminally grasping and excising a tissue sample ;
Turning to the figures, figs. 9 to 14 illustrate a method for endoluminally or laparoscopically grasping and excising a tissue sample from areas in a patient's body. The following description of the method refers both to laparoscopic and endoluminal approaches in which some steps are carried out by means of and under the control of a visualization device (generic term to indicate a visualization device suitable for the chosen approach). In particular, in the case of the endoluminal approach, the steps are carried out and/or are assisted by an endoscope.

The terms "proximal" and "distal" are referred to the surgeon's point of view if not expressly otherwise indicated. The terms "lateral" and "radial" refer to the longitudinal axis L (extending in a proximal-distal direction) of an end effector of a surgical stapling device or to the longitudinal axis of a hollow organ, such as the colon, in which the operation takes place or to the longitudinal axis (proximal-distal direction) of other surgical instruments and devices which will be described in detail below.

In the figures, a portion of target tissue intended to be excised is generally denoted by reference numeral 1. The target tissue 1 might correspond to an entire lesion or other abnormality that need to be examined or removed or might be only a part of such a lesion or abnormality.

Generally, the method according to the present invention comprises the steps of:
- providing traction means 2 having a distal portion 3, suitable for being fixed to at least one portion of the target tissue 1, and a proximal portion,
- introducing the distal portion 3 of the traction means 2 into the patient's body and positioning the distal portion 3 of the traction means 2 close to the target tissue 1, so that at least one portion of the traction means 2 that extends from the distal portion 3 is positioned in the patient's body,
- fixing the distal portion 3 of the traction means 2 to at least one portion of the target tissue 1,
- providing a surgical stapling device 4 having a distal end effector 5 with a window 6 for receiving the target tissue 1 and comprising cutting and stapling means that act through that window 6, wherein the window 6 is defined by a substantially C shaped frame comprising a distal jaw 7 (e.g. a curved anvil) and an opposite proximal jaw 8 (e.g. a staple cartridge device) connected to each other by a lateral shaft 9 as well as a lateral opening 10 opposite the lateral shaft 9,
- introducing the surgical stapling device 4 into the patient's body and positioning the end effector 5 of the surgical stapling device 4 laterally to the target tissue 1 so that the lateral opening 10 is facing towards the target tissue 1,
- moving the end effector 5 laterally or rotating the end effector 5 about its longitudinal axis L so that the target tissue enters the window 6 through the lateral opening 10,
- exerting traction on the traction means 2, thereby pulling an amount of tissue through the window 6,
- cutting and stapling the target tissue 1 by actuating the cutting and stapling means of said surgical stapling device 4 through the window 6.

Finally, all of the devices are withdrawn and the sample fixed to the traction means is removed from the patient's body.

According to an embodiment, the distal portion 3 of the traction means 2 comprises at least one snare 11 and the step of fixing the distal portion 3 of the traction means 2 to at least one portion of the target tissue 1 is carried out by lassoing the snare 11 around the target tissue 1, e.g. a benign adenoma. Moreover, the snare 11 is connected to a distal end 12 of a wire or suture 13 of the traction means 2 to traction the tissue during the rotational movement of the end effector 5 to scoop the target tissue 1 laterally into the window 6 between the opposite distal and proximal jaws 7, 8 and to pull the tissue through the window 6. Indeed, the wire or suture 13 extends between the distal end 12 and a proximal end corresponding to the proximal portion of the traction means 2.

In accordance with an embodiment, the snare 11 is connected to a ring or similar handle (not shown in the figures) formed at a distal end of the wire or suture 13 in order to facilitate traction and pulling of the target tissue 1.

In accordance with an alternative embodiment, the distal portion 3 of the traction means 2 comprises at least one grasper and the step of fixing the distal portion 3 of the traction means 2 to at least one portion of the target tissue 1 is carried out by applying the grasper to the target tissue 1. In this case, the wire or suture 13 and the proximal handle or ring of the traction device can be embodied as described above with reference to the snare traction device.

As shall be described in greater detail, the step of exerting traction on the traction means 2 can comprise the step of extracorporeally pulling the proximal end of the wire or suture 13.

According to a different embodiment of the invention, not shown in figures, the step of exerting traction on the traction means can comprise the step of pulling the proximal end of the wire or suture 13 by means of a further grasping device.

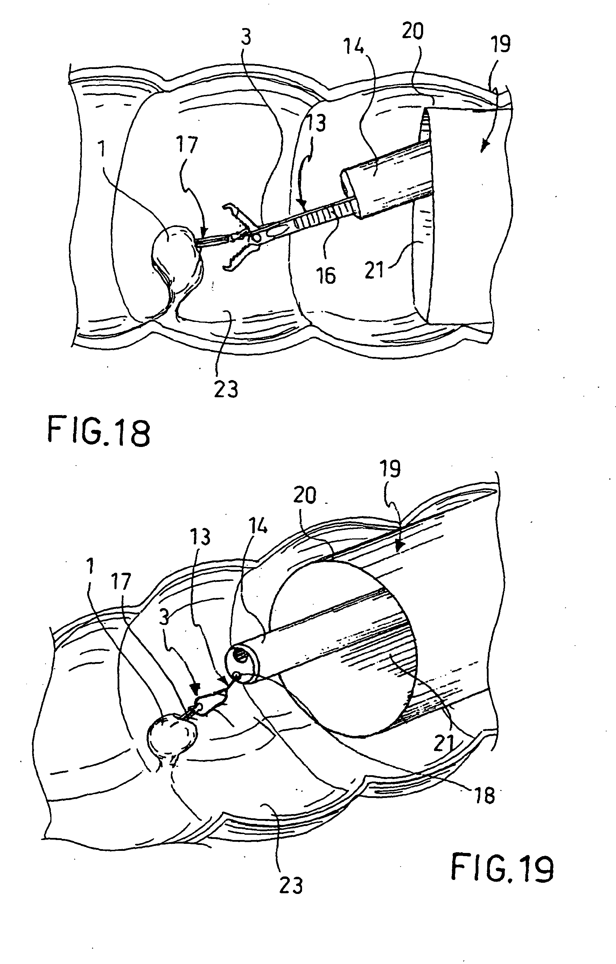

According to an embodiment, the step of applying the distal portion 3 of the traction means 2 to the target tissue 1 and/or the step of exerting a traction on the traction means 2 is carried out under the control of a visualization device 14. In particular, during the step of applying the distal portion 3 of the traction means 2 to the target tissue 1 and/or the step of exerting a traction on said traction means 2, a portion of the wire or suture 13 extends through the visualization device 14 (Figures 10 and 19) by which the proximal end of the wire or suture 13 comes out from the proximal end of the visualization device 14 and the distal end 12 of the wire or suture 13 comes out from a distal end 15 of the visualization device 14.

According to a different embodiment of the invention, not shown in figures, during the step of applying the distal portion 3 of the traction means 2 to the target tissue 1 and/or the step of exerting a traction on said traction means, a portion of said wire or suture 13 extends along an outer surface of a visualization device 14.

In accordance with yet another embodiment, the traction means 2 comprise at least one clip 17 (figures 18, 19). The clip 17 might be connected to the wire or suture 13 whilst the clip 17 is formed in the target tissue 1 and fixed to it.

According to an embodiment, the step of introducing the at least one clip 17 into the patient's body and fixing it in the target tissue 1 is carried out by means of a grasping device 16 that grasp the tissue before fixing the clip to it. For example, the grasping device 16 can be used to introduce and fix the clip 17 with the wire or suture 13 to the target tissue 1 and, after having introduced the surgical stapling device 4, to pull the proximal end of the wire or suture 13 before cutting and stapling the target tissue 1.

According to an embodiment, as shown e.g. in figures 10 and 11 as well as in figures 18 and 19, also the step of introducing the traction means 2 into the patient's body is carried out under the control of a visualization device. In particular, the step of introducing the traction means 2 into the patient's body comprises the step of inserting the traction means 2 through the visualization device 14 outside of the patient's body, and then introducing the visualization device 14 and the traction means 2 into the patient's body. In this case, the wire or suture 13 can be inserted into an instrument channel 18 of the visualization device 14, before or after introducing the visualization device 14, e.g. endoluminally, into the patient's body. According to a different embodiment that is not illustrated, the wire or suture 13 can extend along the outside of the visualization device 14 before introducing the visualisation device 14 into the patient's body.

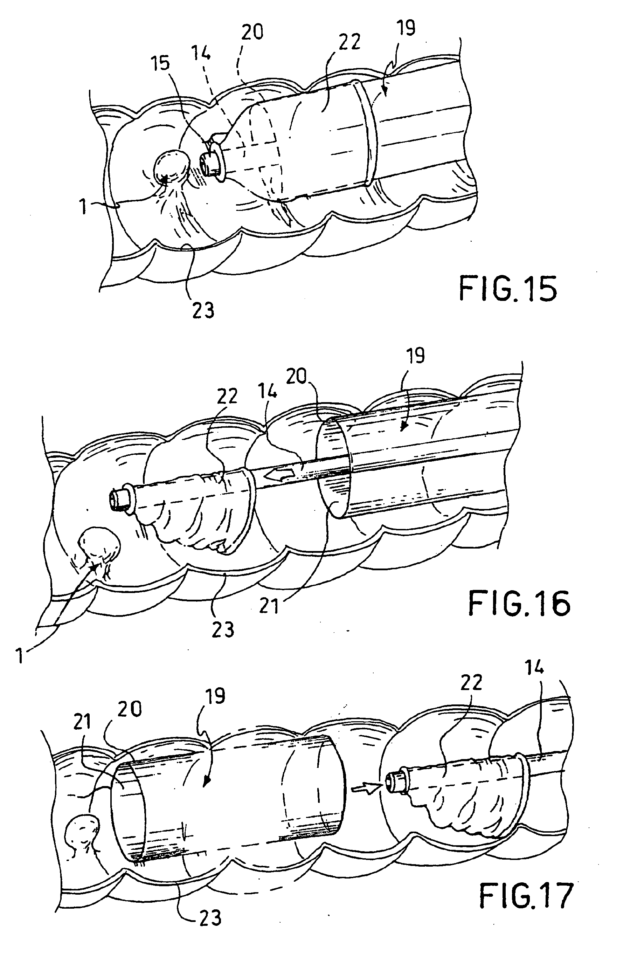

According to a generic embodiment of the invention, schematically illustrated in figures 15 to 19, the present method can also comprise a first step of providing a tubular sheath 19 having a distal end 20 and a proximal end (not shown in the figures). The sheath 19 defines a work channel 21 for receiving in particular the traction means 2 and the surgical stapling device 4. Moreover, the sheath 19 is particularly suitable for protecting the walls from perforations, in particular the walls of the colon when the present method is carried out endoluminally in order to excise a tissue sample of the colon.

According to an embodiment, the sheath 19 is introduced into the patient's body and the distal end 20 of the sheath 19 is positioned close to the target tissue 1 before fixing the traction means 2 to the target tissue 1. Such a sheath 19 can be used in different embodiments of the method, for example having different types of traction means or different types of sheaths. Particularly, the method steps involving the use of a protective sheath can be performed also in connection with the illustration in figures 9 to 14.

The sheath 19 is also introduced into the patient's body by means of a visualization device that can be the same visualization device referred to as 14.

Before introducing the sheath 19 into the patient's body, the visualization device 14 is inserted through the sheath 19, from its proximal end up to and possibly beyond its distal end 20. Thereafter, the visualization device 14 is fixed to the sheath 19 and both are introduced together into the patient's body (Figure 15).

The step of fixing the sheath 19 to the visualization device 14 can be carried out as follows. The visualization device 14 is introduced through the sheath 19 from the proximal end to the distal end of the sheath 19 so that the distal end 15 of the visualization device 14 exits from the distal end 20 of the sheath 19. Then the visualization device 14 is fixed to the sheath 19 by attaching an elastic connection sheath 22 on the distal end 20 of the sheath 19 and on the distal end 15 of the visualization device 14, before introducing the entire assembly of visualization device 14, sheath 19 and elastic connecting sheath 22 into the patient's body (Figure 15).

The visualization device 14 is proximally withdrawn from the sheath 19 before introducing the traction means 2 through the sheath 19 (Figures 16 and 17). By using an elastic connection sheath 22 to fix the sheath 19 to the visualization device 14, the step of withdrawing the visualization device 14 from the sheath 19 may be performed by thrusting the visualization device 14 distally, thereby pulling the elastic connection sheath 22 (which is firmly connected to the distal end 15 of the visualization device 14) from the distal end 20 of the sheath 19 (Figure 16) and subsequently withdrawing the visualization device 14 together with the connecting sheath 22 proximally through the work channel 21 of the sheath 19 (Figure 17).

After the positioning of the sheath 19, the step of introducing the traction means 2 into the patient's body is carried out by passing the traction means 2 through the work channel 21 of the sheath 19 up to the target tissue 1. In order to adequately guide the instruments and at the same time protect the natural duct (e.g. the colon wall) the sheath 19 can be insufflated or can be naturally expanded by introducing the instruments and devices.

Preferably the step of introducing the traction means 2 into the patient's body comprises the step of inserting the distal tissue connection portion 3, e.g. the grasper or the snare, through a visualization device outside of the patient's body, and then of introducing the visualization device and the tissue connection portion 3 into the work channel 21 of the sheath 19 to fix the traction means 2 to the target tissue (Figure 19). The visualization device can be the same visualization device for all of the steps, i.e. the visualization device referred to by reference numeral 14 in the drawings.

The step of introducing the surgical stapling device 4 into the patient's body is generically carried out by inserting the surgical stapling device 4 distally through the work channel 21 of sheath 19 up to its distal end 20. The end effector 5 of the surgical stapling device 4 is then pushed distally out of the sheath 19 and positioned laterally to the target tissue 1 so that the lateral opening 10 of the end effector window 6 is facing towards the target tissue 1. By moving the end effector 5 laterally or by rotating the end effector 5 about its longitudinal axis L so that the target tissue 1 enters the window 6 through the lateral opening 10, the target tissue 1 enters the space between the distal and proximal jaws 7, 8 under very favourable visualization and independently from the volume of the lesion or suspected tissue. During the rotational movement of the surgical stapling device 4 the target tissue 1 can be held in position by pulling the traction means 2. After the target tissue has been laterally introduced (by a scoop - like movement) in between the jaws 7, 8 of the end effector 5, it can be further pulled by means of the traction means 2 in order to obtain the desired tissue positioning in the window 6 of the surgical stapling device 4 prior to perform cutting and stapling through the window 6 in order to excise the portion of target tissue 1.

The step of rotating the end effector about the longitudinal axis L may be carried out by extracorporeally rotating a proximal end portion of the surgical stapling device. An alternative surgical stapling device is provided in which the end effector is rotatably movable with respect to an insertion shaft or force transmitter connecting the end effector to a distal handle and which comprises an end effector rotating mechanism adapted to rotate the end effector about its longitudinal axis L in response to a manual operation of an actuating knob or lever which is arranged at the handle and cooperates with the end effector rotating mechanism. In this case, the step of rotating the end effector about the longitudinal axis L may be carried out by operating the actuating knob or lever.

The step of rotating the end effector about the longitudinal axis L may be carried out by torsionally coupling the end effector to the visualization device and rotating the visualization device about an axis parallel to the longitudinal axis L.

Then all of the devices are withdrawn from the patient's body and the excised sample is taken out by means of the traction means 2.

As has already been mentioned, the hitherto described method is particularly suitable for being carried out according to an endoluminal approach, using natural or artificial orifices. However, the method described can also be carried out according to a laparoscopic approach. With reference to all of the embodiments described, the step of fixing the traction means 2 to at least one portion of the target tissue 1 can comprise the step of fixing a plurality of combined or single clips and/or snares and/or graspers of the traction means to the target tissue 1. Each clip and/or snare and/or grasper of said plurality of combined or single clips, snares or graspers is connected to a wire or suture 13 to hold the target tissue 1 during the rotational movement of the end effector 5 to laterally embrace the target tissue 1 and position it in the window 6 and, possibly, to subsequently pull the target tissue 1 further through the window 6 prior to cutting and stapling.

Said clips or graspers are fixed along a border of the portion of the target tissue. Alternatively, at least one clip or grasper is fixed directly to the portion of the target tissue 1. In case of pedunculated polyps, a snare is preferably lassoed around the stalk of the polyp.

With reference to all of the embodiments described, the method of using the present invention can comprise the step of highlighting the target tissue 1 through marks or tattoos and, whilst a traction is exerted on the traction means, of monitoring the amount of tissue positioned in and pulled through the window 6 of the surgical stapling device 4 by checking the position of the marks or of the tattoos.

A further, more specific illustrative example of an endoluminal excision of a benign adenoma at the colon wall of a patient will be given below with reference to figures 9 to 14.

In a first step of the method (Figure 9), a colonoscope 14 is introduced transanally through the entire colon 23 up to the caecum and then slowly withdrawn proximally to visualize the colon wall in order to individualize and locate colonic lesions. In the illustrative example of figure 9, a benign pedunculated adenoma (target tissue 1) is identified.

In order to hold and traction the adenoma during the following steps of the method, an endoscopic snare 2, 11, 13 is passed through the instrument channel 18 of the colonoscope 14 until it exits the colonoscope 14 at its distal end 15 (Figure 10). The snare 11 is positioned about the adenoma and lassoed around its stalk 24 (even though the figures show a schematically wide open snare, it is of course intended to tighten the snare 11 around the lesion in order to fixate it properly to the tissue). After having the snare 11 attached to the adenoma, the surgical stapling device 4 is transanally introduced into the colon and advanced along the colonoscope 14 up to its distal end 15. During transanal introduction and distal advancing of the surgical stapling device 4, the distal end effector 5 is held in a fully closed configuration, that is the distal and proximal jaws 7, 8 are completely approximated (thereby closing the window 6) in order not to damage rectal and colon wall tissue during introduction.

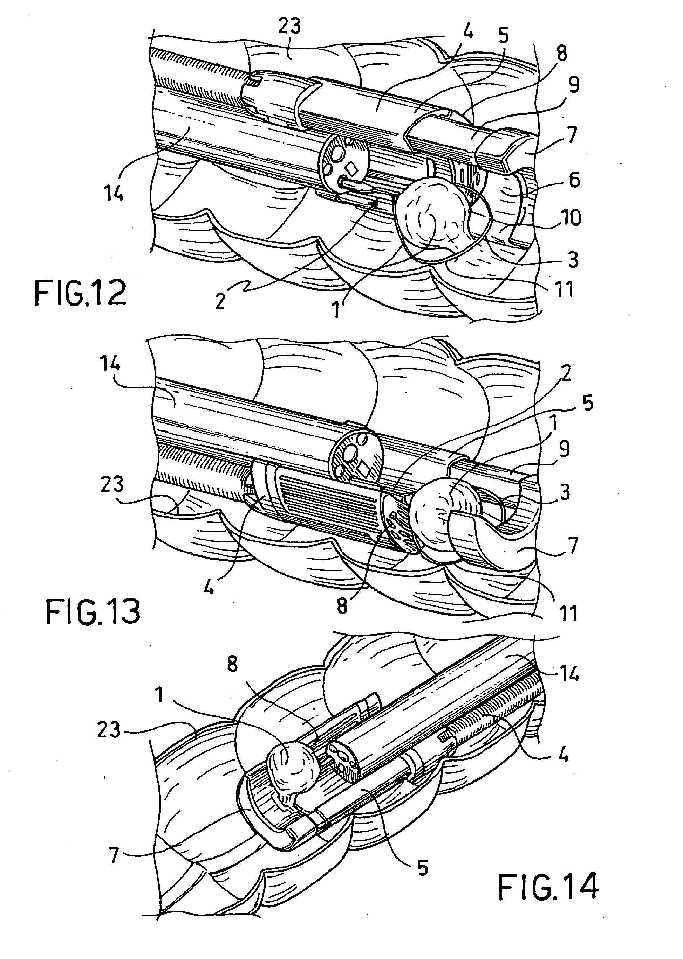

The jaws 7, 8 of the end effector 5 are then fully opened thereby opening the window 6 therebetween and also widening the lateral opening 10. Under continuous endoscopic visualization by the colonoscope 14, the end effector 5 is now advanced distally and positioned laterally to the previously grasped adenoma (target tissue 1) so that the lateral opening 10 directly faces the portion of target tissue 1 intended to be placed in the window 6 between the distal and proximal jaws 7, 8 (Figure 12). The end effector 5 is then rotated about its longitudinal axis L in order to embrace (e.g. in a scoop like manner) the adenoma from the side so that it enters the window 6 through the lateral opening 10 (Figure 13).

With the adenoma placed in the window 6 of the end effector 5, it can now be tractioned from outside the body by means of the snare 11 and the associated wire 13 until the desired amount of tissue volume is captured.

Then the jaws 7, 8 are approximated to clamp and compress the target tissue along the intended cutting and staple line. After a certain period (of e.g. about 5 to 10 seconds) sufficient to squeeze fluids out of the staple line region of the target tissue, a cutting and staple driving mechanism of the end effector 5 is actuated to apply the staples to the tissue and to cut the latter adjacent the stapled seam, thereby excising the colonic adenoma (Figure 14).

Then, the jaws 7, 8 are fully opened and the stapled formation is visually assessed by means of the colonoscope 14.

Finally, the colonoscope 14, the surgical stapling device 4 and the traction means 2 holding the excised lesion are proximally withdrawn from the target site and out of the body of the patient.

Those skilled in the art will have appreciated from the foregoing description that the method of using the invention reduces the invasiveness with respect to known methods and provides an improved visualization during detection, grasping and positioning of the tissue in the end effector of the surgical stapling device in an endoluminal or laparoscopic approach. Moreover, said method allows to increase the amount of target tissue that can be grasped and excised.

According to the invention and in order to facilitate the implementation of the above described method, the surgical stapling device 4 comprises, in its distal end region, an end effector 5, particularly a staple fastening assembly, and in its proximal end region, a handle (not shown in the figures). The handle and the end effector 5 are connected via one or more, e.g. two elongate flexible force transmitters 25 used for transmitting forces and/or movements from the proximal handle to the distal end effector 5 in order to position (e.g. advance and rotate) the end effector and to perform the stapling and cutting functions of the stapling device 4.

The main components of the end effector 5 are a cartridge device (proximal jaw 8), which contains several curved open rows of staples as well as a knife or cutting edge, and a curved anvil (distal jaw 7), which has a staple forming face and is adapted to cooperate with the cartridge device to form the ends of the staples expelled from the cartridge device when the stapling device 4 is "fired".

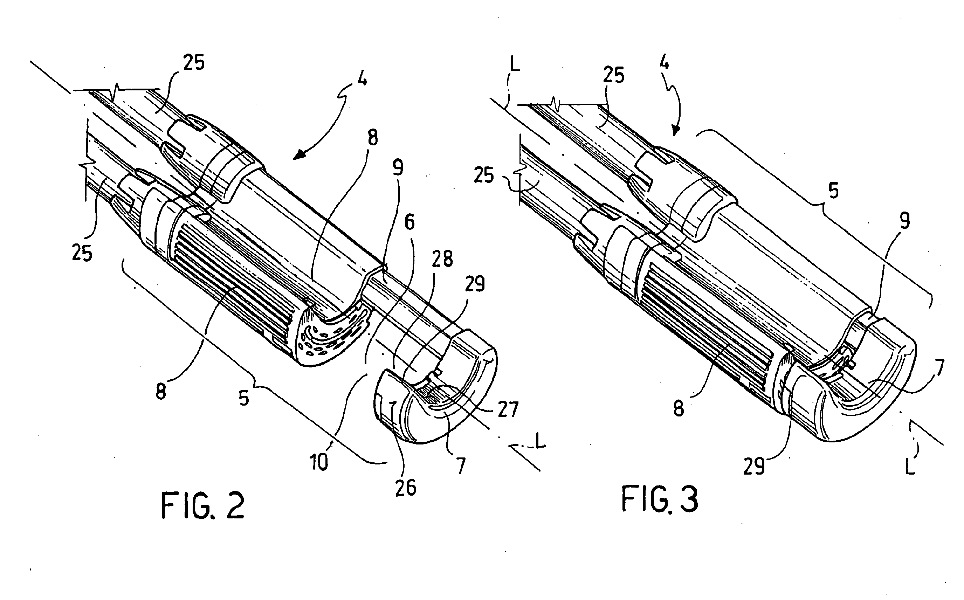

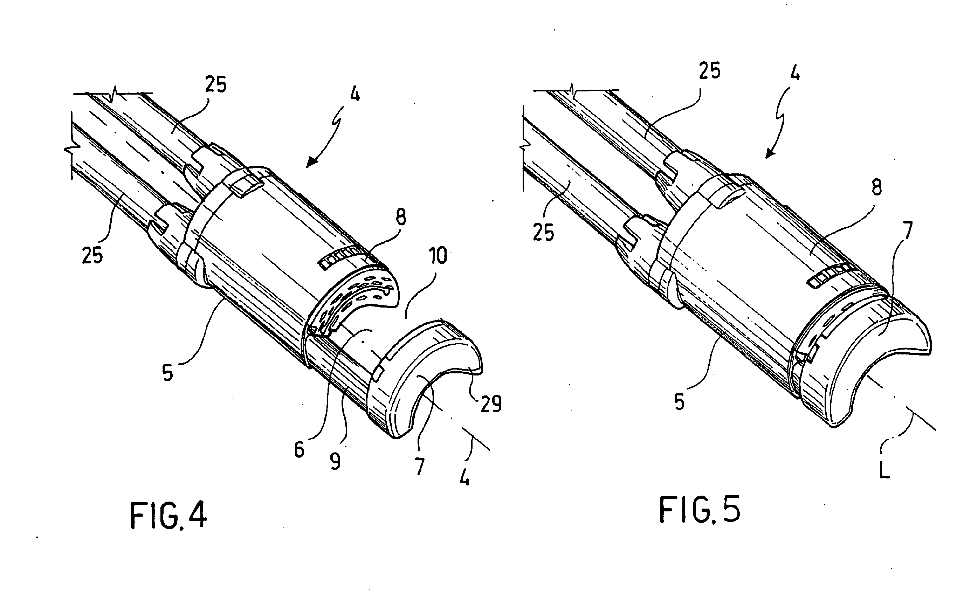

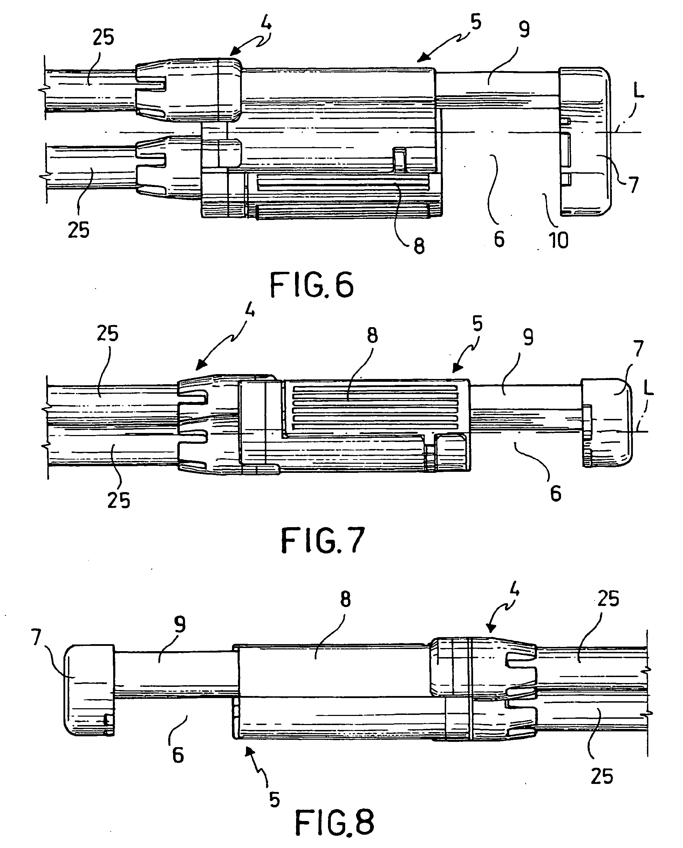

The anvil 7 can be moved with respect to the cartridge device 8 in a parallel relationship, i.e. in a direction parallel to the longitudinal axis L of the end effector 5. In the views of Fig. 2, 4 and 6, the anvil 7 is spaced apart from the cartridge device 8, while in Fig. 3 and 5, the same anvil 7 has been entirely moved towards the cartridge device 8. The mechanism and its components of the stapling device 4 used for moving anvil 7 relative to cartridge device 8 are generally called moving device, whereas the mechanism and the components used for advancing the staples are generally called staple driving device.

For the sake of simplicity, in the present description the above mentioned moving device and staple driving device will be referred to as a whole with the term "actuating mechanism" of the surgical stapling device 4. As the detailed description of one possible embodiment of such an actuating mechanism is concerned, such a description is disclosed in the applicant's co-pending international patent application WO 2006/027014, figures 4 to 15 and the corresponding parts of the description which are herewith included for reference.

In accordance with the invention, the entire end effector 5 is curved about the longitudinal (proximal - distal) axis L so that its shape adapts to the wall of natural ducts, such as the colon wall, without obstructing access space and visualization.

A lateral end of the anvil 7 and a corresponding lateral end of the cartridge device 8 are connected by the longitudinally extending lateral shaft 9 so that a substantially C shaped frame is formed which defines the window 6 for receiving the target tissue 1 and the lateral opening 10 opposite the lateral shaft 9 and configured to provide lateral access to the window 6. The cutting and stapling means act along the theoretical curved surface of the aperture of window 6 in order to act exactly on the target tissue placed in that window 6.

In other words, the lateral opening 10 provides a substantially tangentially or circumferentially oriented access aperture to the window 6, while the window 6 itself provides a substantially radially oriented (with respect to the longitudinal axis L) through opening for receiving the target tissue 1 during stapling and cutting.

According to the invention, a lateral end 28 of the curved anvil 7 opposite the lateral shaft 9 is radially tapered from a radially external side 26 towards a radially internal side 27 so that it defines an edge 29 extending substantially parallel to the longitudinal axis L of the end effector 5. The edge 29 is preferably blunt and facilitates the above mentioned rotational scooping of the target tissue 1 in order to place it in the window 6 between the anvil 7 and the cartridge device 8.

Thanks to the particular shape and structural configuration of the surgical stapling device 4, it is adapted to be slit along an endoscope during advancement to the target tissue 1, to perform the above mentioned rotational movement about the longitudinal axis L in order to place the target tissue 1 between the jaws embodied by the anvil and cartridge device and to keep the endoluminal operational site clear, thereby providing a good visualization and access.

Moreover, thanks to the lateral opening 10, the traction means need not be inserted through the window 6 prior to grasping the tissue or prior to insertion of the traction means or of the surgical stapler into the body of the patient.

## Claims

1. Surgical device for endoluminally or laparoscopically grasping and excising a target tissue (1) sample from areas in a patient's body, comprising a surgical stapling device (4) having a distal staple fastening assembly (5) connected via one or more force transmitters (25) to a proximal handle,
wherein the staple fastening assembly (5) comprises:
- a cartridge device (8) containing one or more curved open rows of staples as well as a knife,
- a curved anvil (7) having a staple forming face adapted to cooperate with the cartridge device (8) to form the ends of the staples expelled from the cartridge device (8),
wherein the anvil (7) is movable with respect to the cartridge device (8) along a longitudinal axis (L) of the staple fastening assembly (5),
wherein the staple fastening assembly (5) is curved about the longitudinal axis (L) and forms two opposite lateral ends,
**characterized in that** said one or more force transmitters (25) are flexible, **in that** only one lateral end of the anvil (7) is connected to a corresponding lateral end of the cartridge device (8) by a longitudinally extending lateral shaft (9), so that a substantially C shaped frame is formed which defines a window (6) for receiving the target tissue (1) and a lateral opening (10) opposite the lateral shaft (9) configured to provide lateral access to the window (6) and **in that** a free lateral end (28) of the curved anvil (7) opposite said lateral shaft (9) is radially tapered from a radially external side (26) towards a radially internal side (27) so that it defines an edge (29) extending substantially parallel to the longitudinal axis (L).

2. Surgical device according to claim 1, wherein said lateral opening (10) is configured to provide a substantially tangentially or circumferentially oriented access aperture to the window (6) and the window (6) itself provides a substantially radially oriented through opening for receiving the target tissue 1 with respect to the longitudinal axis L.

3. Surgical device according to claim 1 or 2, wherein said edge (29) is blunt.

4. Surgical device according to any one of the preceding claims, wherein the staple fastening assembly (5) is rotatably movable with respect to the force transmitter (25) and the surgical stapling device (4) comprises a rotating mechanism adapted to rotate the staple fastening assembly (5) about its longitudinal axis (L) in response to an actuation of an actuating knob or lever which is arranged at the handle and which cooperates with said rotating mechanism.

5. Surgical device according to any one of the preceding claims, comprising traction means (2) with a distal tissue connection portion (3) and a wire or suture (13) connected to said distal tissue connection portion (3).

6. Surgical device according to claim 5, wherein said distal tissue connection portion (3) is selected from the group comprising:
- a snare (11),
- a grasping device (16),
- a clip (17).

7. Surgical device according to any one of the claims 5 or 6, comprising a visualization device (14), such as an endoscope or laparoscope, having an instrument channel (18) adapted to receive the traction means (2).

8. Surgical device according to any one of the preceding claims, comprising a protective sheath (19) adapted to be inserted in a natural duct of the patient's body and defining a work channel (21) adapted to receive the surgical stapling device (4) and a visualization device (14) .

## Patentansprüche

1. Chirurgische Vorrichtung für endoluminales oder laparoskopisches Greifen und Herausschneiden einer Probe eines Zielgewebes (1) aus Bereichen in einem Körper eines Patienten, umfassend eine chirurgische Klammervorrichtung (4), die eine distale Klammerbefestigungsanordnung (5) aufweist, die mittels einer oder mehrerer Kraftübertragungseinrichtungen (25) mit einem proximalen Griff verbunden ist,
wobei die Klammerbefestigungsanordnung (5) umfasst:
- eine Patronenvorrichtung (8), die eine oder mehrere gekrümmte offene Klammerreihen, als auch ein Messer beinhaltet,
- einen gekrümmten Amboss (7), der eine Klammer-formende Fläche aufweist, die angepasst ist, mit der Patronenvorrichtung (8) zusammen zu wirken, um die Enden der Klammern, die aus der Patronenvorrichtung (8) ausgestoßen sind, zu formen,
wobei der Amboss (7) bezüglich der Patronenvorrichtung (8) entlang einer Längsachse (L) der Klammerbefestigungsanordnung (5) bewegbar ist,
wobei die Klammerbefestigungsanordnung (5) um die Längsachse (L) gekrümmt ist und zwei entgegengesetzte seitliche Enden bildet,
**dadurch gekennzeichnet,**
**dass** die eine oder die mehrere Kraftübertragungseinrichtungen (25) flexibel sind,
**dass** nur ein seitliches Ende des Ambosses (7) mit einem dazugehörigen seitlichen Ende der Patronenvorrichtung (8), durch einen sich in Längsrichtung erstreckenden Seitenschaft (9) verbunden ist, so dass ein im Wesentlichen C-förmiger Rahmen gebildet wird, der ein Fenster (6) zum Aufnehmen des Zielgewebes (1) und eine dem Seitenschaft (9) entgegengesetzte Seitenöffnung (10) definiert, die konfiguriert ist, einen seitlichen Zugriff zum Fenster (6) bereit zu stellen, und
**dass** ein freies seitliches Ende (28) des gekrümmten Ambosses (7), das dem Seitenschaft (9) entgegengesetzt ist, radial konisch von einer radial äußeren Seite (26) zu einer radial inneren Seite (27) hin abnimmt, so dass es einen Rand (29) definiert, der sich im Wesentlichen parallel zur Längsachse (L) erstreckt.

2. Chirurgische Vorrichtung nach Anspruch 1, wobei die Seitenöffnung (10) konfiguriert ist, einen im Wesentlichen tangential oder in der Umfangsrichtung orientierten Zugriffsdurchgang zum Fenster (6) bereit zu stellen und das Fenster (6) selbst eine im Wesentlichen bezüglich der Längsachse (L) radial orientierte Durchgangsöffnung zum Aufnehmen des Zielgewebes (1) bereitstellt.

3. Chirurgische Vorrichtung nach Anspruch 1 oder 2,
wobei der Rand (29) stumpf ist.

4. Chirurgische Vorrichtung nach einem der vorhergehenden Ansprüche,
wobei die Klammerbefestigungsanordnung (5) bezüglich der Kraftübertragungseinrichtung (25) drehbar beweglich ist und die chirurgische Klammervorrichtung (4) eine Dreheinrichtung umfasst, die angepasst ist, die Klammerbefestigungsanordnung (5) um ihre Längsachse (L) als Antwort auf eine Betätigung eines Betätigungsknopfes oder Hebels, welcher an dem Griff angeordnet ist und welcher mit der Dreheinrichtung zusammen wirkt, zu drehen.

5. Chirurgische Vorrichtung nach einem der vorhergehenden Ansprüche, umfassend Zugmittel (2), mit einem distalen Gewebe-Verbindungsabschnitt (3) und einem Draht oder einer Naht (13), der/die mit dem distalen Gewebe-Verbindungsabschnitt (3) verbunden ist.

6. Chirurgische Vorrichtung nach Anspruch 5,
wobei der distale Gewebe-Verbindungsabschnitt (3) ausgewählt ist aus der Gruppe umfassend:
- eine Schlinge (11),
- eine Greifvorrichtung (16),
- eine Klammer (17).

7. Chirurgische Vorrichtung nach einem der Ansprüche 5 oder 6,
umfassend eine Visualisierungsvorrichtung (14), wie beispielsweise ein Endoskop oder Laparoskop, die einen Instrumentenkanal (18) aufweist, der angepasst ist, die Zugmittel (2) aufzunehmen.

8. Chirurgische Vorrichtung nach einem der vorhergehenden Ansprüche, die eine Schutzhülle (19) umfasst, welche angepasst ist, in einen natürlichen Ductus des Körpers des Patienten eingeführt zu werden, und die einen Arbeitskanal (21) definiert, der angepasst ist, die chirurgische Klammervorrichtung (4) und eine Visualisierungsvorrichtung (14) aufzunehmen.

## Revendications

1. Dispositif chirurgical pour saisir et exciser par voie intraluminale ou laparoscopique un échantillon de tissu cible (1) depuis des régions dans le corps d'un patient, comprenant un dispositif d'agrafage chirurgical (4) ayant un ensemble de fixation d'agrafe distal (5) relié via un ou plusieurs transmetteurs de force (25) à une poignée proximale,
dans lequel l'ensemble de fixation d'agrafe (5) comprend :
- un dispositif formant cartouche (8) contenant une ou plusieurs rangées ouvertes incurvées d'agrafes, ainsi qu'un couteau,
- une enclume incurvée (7) ayant une face de formage d'agrafe adaptée à coopérer avec le dispositif formant cartouche (8) pour former les extrémités des agrafes expulsées hors du dispositif formant cartouche (8),
dans lequel l'enclume (7) est mobile par rapport au dispositif formant cartouche (8) le long d'un axe longitudinal (L) de l'ensemble de fixation d'agrafe (5),
dans lequel l'ensemble de fixation d'agrafe (5) est incurvé autour de l'axe longitudinal (L) et forme deux extrémités latérales,
**caractérisé en ce que** lesdits un ou plusieurs transmetteurs de force (25) sont flexibles **en ce qu'**une seule extrémité latérale de l'enclume (7) est reliée à une extrémité latérale correspondante du dispositif formant cartouche (8) par un arbre latéral (9) s'étendant longitudinalement, de sorte qu'il se forme un cadre sensiblement en forme de C qui définit une fenêtre (6) pour recevoir le tissu cible (1) et une ouverture latérale (10) à l'opposé de l'arbre latéral (9), configuré pour donner un accès latéral à la fenêtre (6), et **en ce qu'**une extrémité latérale libre (28) de l'enclume incurvée (7) à l'opposé dudit arbre latéral (9) est effilée radialement, depuis un côté radialement extérieur (26) vers un côté radialement intérieur (27) de sorte qu'elle définit une arête (29) s'étendant sensiblement parallèlement à l'axe longitudinal (L).

2. Dispositif chirurgical selon la revendication 1, dans lequel ladite ouverture latérale (10) est configurée pour donner un accès orienté sensiblement tangentiellement ou circonférentiellement vers la fenêtre (6), et la fenêtre elle-même (6) constitue une ouverture traversante orientée sensiblement radialement pour recevoir le tissu cible (1) par rapport à l'axe longitudinal (L).

3. Dispositif chirurgical selon la revendication 1 ou 2, dans lequel ladite arête (29) est émoussée.

4. Dispositif chirurgical selon l'une quelconque des revendications précédentes, dans lequel l'ensemble de fixation d'agrafe (5) est mobile en rotation par rapport au transmetteur de force (25) et le dispositif d'agrafage chirurgical (4) comprend un mécanisme rotatif adapté à faire tourner l'ensemble de fixation d'agrafe (5) autour de son axe longitudinal (L) en réponse à un actionnement d'un bouton d'actionnement ou d'un levier d'actionnement qui est agencé au niveau de la poignée et qui coopère avec ledit mécanisme rotatif.

5. Dispositif chirurgical selon l'une quelconque des revendications précédentes, comprenant des moyens de traction (2) avec une portion de liaison de tissu distale (3) et un fil ou une suture (13) relié(e) à ladite portion de liaison de tissu distale (3).

6. Dispositif chirurgical selon la revendication 5, dans lequel ladite portion de liaison de tissu distale (3) est choisie parmi le groupe comprenant :
- un noeud coulant (11),
- un dispositif de saisie (16),
- une pince (17).

7. Dispositif chirurgical selon l'une quelconque des revendications 5 ou 6, comprenant un dispositif de visualisation (14), comme un endoscope ou un laparoscope, ayant un canal à instruments (18) adapté à recevoir les moyens de traction (2).

8. Dispositif chirurgical selon l'une quelconque des revendications précédentes, comprenant une gaine protectrice (19) adaptée à être introduite dans un conduit naturel du corps du patient et définissant un canal de travail (21) adapté à recevoir le dispositif d'agrafage chirurgical (4) et un dispositif de visualisation (14).
